# EUROPEAN PATENT APPLICATION

(11) **EP 4 813 451 A1**
(43) Date of publication of application: **30.09.2026**
(21) Application number: 26167110.1
(22) Date of filing: 24.03.2026
(51) Int. Cl.: A61N 5/10

(54) **LOCAL DOSE HETEROGENEITY METRIC FOR SPATIALLY FRACTIONATED RADIOTHERAPY**

(30) Priority: 27.03.2025 US 202519091826
(71) Applicant: Siemens Healthineers International AG, 6312 Steinhausen (CH)
(72) Inventor: LANSONNEUR, Pierre, 92357 Le Plessis-Robinson (FR)
(74) Representative: Mathisen & Macara LLP

(57) **Abstract**

A method is described for planning and administering a spatially fractionated radiotherapy treatment plan. The method may include receiving the treatment plan that includes a heterogenous radiotherapy dose that has a plurality of local maximum doses 304. 306 and a plurality of local minimum doses 307, 308. The method may include generating an upper heterogenic curve 320 based at least in part on the plurality of local maximum doses and generating a lower heterogenic curve 322 based at least in part on the plurality of local minimum doses. The method may include determining a dose envelope bounded by the upper heterogenic curve and the lower heterogenic curve. The method may include generating a local dose contrast 318 based on the upper and lower heterogenic curves of the dose envelope.

## Description

### TECHNICAL FIELD

This application relates generally to the planning (and optional execution) of radiotherapy treatment.

### BACKGROUND

Radiation therapy is a widely used treatment for cancer, traditionally delivering a homogeneous radiation dose to a target volume (e.g., a tumor) while minimizing exposure to surrounding healthy tissue. The effectiveness of traditional radiation therapy has largely depended on precise dose delivery to the target volume while reducing toxicity to organs at risk.

Certain radiotherapy treatments, such as spatially fractionated radiotherapy (SFRT), have shown a potential to improve therapeutic outcomes by delivering non-homogenous radiation doses to target volumes. Unlike conventional radiation therapy that delivers a homogenous radiation dose, SFRT introduces a heterogeneous dose radiation with alternating dose peaks and valleys. These methods improve tissue recovery and reduce side effects that are experienced in traditional homogenous radiation therapies. However, current treatment planning methodologies lack adequate tools to locally quantify and optimize the dynamic nature of the local dose heterogeneity of SFRT during the planning and administration of SFRT, resulting in delayed treatment and inefficient administration of the radiotherapy.

### SUMMARY

For the aforementioned reasons, there is a need for improved methods and systems for planning and optionally administering SFRT, including a need for a local dose heterogeneity metric that is computationally stable, localized, clinically relevant, and enables precise dose assessment and optimization within SFRT treatment planning and administration. It is understood that the implementations of the systems and methods described herein may satisfy more, fewer, or different needs than those improvements described above without departing from the scope of the descriptions herein.

The current/conventional methods for planning and administering SFRT face technical challenges due to the lack of accurate and reliable metrics for quantifying and optimizing local dose heterogeneity within a treatment volume. SFRT aims to deliver an inhomogeneous dose distribution, using alternating high-dose peaks and low-dose valleys to enhance tumor destruction while sparing healthy tissue. However, the traditional peak-to-valley dose ratio has proven to be inadequate, as it becomes numerically unstable when the valley dose is near zero, leading to imprecise measurements that hinder treatment planning. This challenge prevents clinicians from optimally adjusting treatment parameters at a local level, causing delays, inefficiencies, and potentially compromising treatment effectiveness.

Moreover, conventional computer models that utilize traditional methods for evaluating radiotherapy treatment plans are computationally inefficient and demand extensive computing resources due to their reliance on brute-force dose calculations and global optimization techniques. These methods often require high-resolution dose simulations over the entire treatment volume, leading to excessive processing times and memory consumption. Furthermore, conventional peak-to-valley dose ratio calculations struggle with numerical instability, particularly in regions where valley doses approach zero, necessitating additional computational corrections that further increase processing overhead. Without an optimized framework for local dose contrast evaluation, these models must iteratively adjust treatment parameters across large datasets, resulting in prolonged optimization cycles, which results in high processing power requirements and delayed output of results.

The disclosed methods and systems address these technical inefficiencies by introducing a computationally stable and localized dose heterogeneity metric that enables precise and efficient evaluation of spatially fractionated radiotherapy (SFRT) treatment plans. Instead of relying on global dose calculations or unstable peak-to-valley dose ratio methods, the system interpolates between local maximum and minimum dose points to generate upper and lower heterogenic curves, defining a dose envelope that can be rapidly analyzed for local dose contrast. This approach may reduce computational overhead by focusing calculations only on clinically relevant regions, eliminating unnecessary full-volume simulations, which results in shorter execution times and the use of less computing processing power.

By integrating this local dose contrast metric into an automated treatment planning system, the invention may enable real-time optimization of SFRT parameters, allowing for faster adjustments to treatment plans while maintaining accuracy and minimizing computing resource consumption.

In at least one embodiment of the descriptions here, a local dose heterogeneity metric for evaluating a local dose contrast between a dose peak and dose valley is used to determine localized heterogeneity of the SFRT plan at localized positions within a treatment area. The local dose contrast is generated for a desired position by (i) interpolating an upper dose between local maximum doses, (ii) interpolating a lower dose between local minimum doses, and (iii) determining a contrast between the interpolated upper dose and the interpolated lower dose. This process is repeated to determine a local dose contrast for each position within a dose envelope that is bounded by the interpolated upper dose and the interpolated lower dose.

These local dose contrasts are combined to generate a heterogeneity heatmap of the SFRT treatment plan. The heterogeneity heatmap is overlaid on a diagnostic image of a tumor and surrounding organs at risk. By overlaying the heterogeneity heatmap on the diagnostic image, the SFRT may be more efficiently evaluated and adjusted to improve efficacy of the treatment while minimizing damage to neighboring tissues. Overlaying the heterogeneity heatmap on the diagnostic image allows physicians and clinicians quickly and accurately determine biological effects on the target tumor and surrounding organs at risk.

In one aspect, the present invention provides a method for adjusting a spatially fractionated radiotherapy treatment plan, as defined in claim 1. In another aspect, the present invention provides a system for adjusting a spatially fractionated radiotherapy treatment plan, as defined in claim 2. In a further aspect, the present invention provides a computer-readable, non-transitory storage medium containing instructions for adjusting a spatially fractionated radiotherapy treatment plan and, when executed by one or more processors, cause the one or more processors to perform a method for adjusting a spatially fractionated radiotherapy treatment plan, as defined in claim 10. Optional features are specified in the dependent claims.

In an embodiment, a method may include: receiving a radiotherapy treatment plan to administer to a target tissue, the radiotherapy treatment plan having a heterogeneous dose including: a plurality of local maximum doses, and a plurality of local minimum doses; generating
(i) an upper heterogenic curve based at least in part on the plurality of local maximum doses and
(ii) a lower heterogenic curve based at least in part on the plurality of local minimum doses; determining a dose envelope of the radiotherapy treatment plan bounded by the upper heterogenic curve and the lower heterogenic curve; and generating a local dose contrast of the dose envelope at a position within the dose envelope.

In response in part to the local dose contrast exceeding a threshold, a treatment parameter of the radiotherapy treatment plan may be adjusted to reduce the local dose contrast.

The method may further include generating a local dose contrast distribution including a plurality of local dose contrasts; and generating a heatmap of a heterogeneity of the radiotherapy treatment plan based on the local dose contrast distribution.

The local dose contrast may be generated based in part on the upper heterogenic curve and the lower heterogenic curve at the position.

Generating the upper heterogenic curve may include interpolating heterogeneous dose between adjacent local maximum doses within the plurality of local maximum doses.

The method may include linearly interpolating the heterogeneous dose between the adjacent local maximum doses within the plurality of local maximum doses.

The method may include receiving a diagnostic image of the target tissue; displaying the diagnostic image of the target tissue on a display; and overlaying the heatmap on the diagnostic image of the target tissue.

The method may include adjusting one or more operating parameters of a radiotherapy machine in accordance with the radiotherapy treatment plan; and transmitting, by the one or more processors, instructions to the radiotherapy machine to administer the radiotherapy treatment plan to the target tissue.

In an embodiment, a system may include one or more processors; and a computer-readable, non-transitory storage medium containing instructions that, when executed by the one or more processors, cause the one or more processors to perform a method including: receiving a radiotherapy treatment plan to administer to a target tissue, the radiotherapy treatment plan having a heterogeneous dose including: a plurality of local maximum doses, and a plurality of local minimum doses; generating (i) an upper heterogenic curve based at least in part on the plurality of local maximum doses and (ii) a lower heterogenic curve based at least in part on the plurality of local minimum doses; determining a dose envelope of the radiotherapy treatment plan bounded by the upper heterogenic curve and the lower heterogenic curve; and generating a local dose contrast of the dose envelope at a position within the dose envelope.

In an embodiment, a computer-readable, non-transitory storage medium containing instructions that, when executed by one or more processors, cause the one or more processors to perform a method including: receiving a radiotherapy treatment plan to administer to a target tissue, the radiotherapy treatment plan having a heterogeneous dose including: a plurality of local maximum doses, and a plurality of local minimum doses; generating (i) an upper heterogenic curve based at least in part on the plurality of local maximum doses and (ii) a lower heterogenic curve based at least in part on the plurality of local minimum doses; determining a dose envelope of the radiotherapy treatment plan bounded by the upper heterogenic curve and the lower heterogenic curve; and generating a local dose contrast of the dose envelope at a position within the dose envelope.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2** is a block diagram of a method for planning and administering a radiotherapy treatment plan, according to an embodiment.
**FIG. 3** illustrates a dose envelope of a radiotherapy dose treatment, according to an embodiment.
**FIG. 4** illustrates a dose distribution from a radiotherapy treatment beam in a patient, according to an embodiment.
**FIG. 5** illustrates various distribution views of a radiotherapy treatment plan, according to an embodiment.
**FIG. 6** illustrates various distribution views of a radiotherapy treatment plan, according to an embodiment.
**FIG. 7** is a block diagram of a method for an automated radiation therapy treatment planning process, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein, as illustrated here, which would occur to a person skilled in the relevant art and having possession of this disclosure, are to be considered within the scope of the embodiments, methods, and/or systems described herein.

Unlike conventional radiotherapy, which delivers a homogeneous dose to a tumor or other target tissue, SFRT introduces a heterogeneous dose distribution, characterized by alternating high-dose (peak) and low-dose (valley) regions. The goal is to maximize tumor destruction while allowing normal tissues to recover more effectively. The treatment can be delivered through various methods. GRID therapy uses a perforated collimator or blocking grid to deliver parallel, high-dose radiation beams, creating a checkerboard-like dose pattern that is useful for treating large tumors. Lattice therapy is a more advanced form of SFRT that optimizes dose placement in three dimensions, creating localized high-dose islands within a low-dose background, allowing for precise tumor targeting using techniques like Intensity-Modulated Radiation Therapy (IMRT) or Volumetric Modulated Arc Therapy (VMAT). Microbeam and Minibeam Radiation Therapy (MRT/MBRT) utilize extremely narrow radiation beams to achieve an even finer dose distribution, demonstrating potential for improved tumor control with minimal damage to normal tissue, particularly in preclinical studies.

SFRT takes advantage of differential tumor and normal tissue responses to radiation. High-dose peak regions cause direct tumor cell death, triggering an immune response and vascular damage that further compromises tumor viability. Meanwhile, low-dose valley regions allow healthy tissue to repair and regenerate, reducing overall radiation toxicity and side effects. Additionally, the bystander effect suggests that cells in low-dose regions may still undergo damage due to signals from adjacent high-dose areas, further enhancing tumor destruction.

To ensure proper dose delivery, imaging techniques such as CT, MRI, and PET help define the tumor volume and surrounding structures. Treatment Planning Systems (TPS) optimize beam arrangement and dose modulation to create the correct spatial fractionation pattern. Local dose metrics, such as the local contrast-based heterogeneity metric, improve SFRT evaluation compared to traditional peak-to-valley dose ratio (PVDR) calculations, which can be unstable and less precise.

SFRT offers several advantages over conventional radiotherapy. It enables higher tumor control probability by allowing dose escalation to resistant tumors while sparing normal tissues. The presence of valley regions permits healthy cells to recover, leading to fewer side effects compared to homogeneous dose treatments. SFRT may also stimulate systemic anti-tumor immune responses, increasing overall treatment effectiveness. This technique is particularly beneficial for bulky, hypoxic tumors that are difficult to treat with standard radiation approaches.

As an innovative radiotherapy approach, SFRT harnesses spatial dose heterogeneity to enhance cancer treatment efficacy. By integrating modern treatment planning techniques, advanced imaging, and optimized dose metrics, SFRT provides a promising alternative for improving tumor control while reducing side effects, making it a valuable addition to modern radiation oncology.

The level of heterogeneity (e.g., the difference between the high-dose peak and low-dose valley at a point) at a position is used to determine the effectiveness of the SFRT at that position. This heterogeneity may be measured with a local dose contrast metric. The local dose metric may be used during the planning, administration, and review of SFRT to provide clinicians and physicians localized values of heterogeneity of an SFRT plan to (i) accelerate administration of the SFRT plan, (ii) improve outcomes of the SFRT plan, and/or (iii) increase the efficiency of computing the SFRT plan.

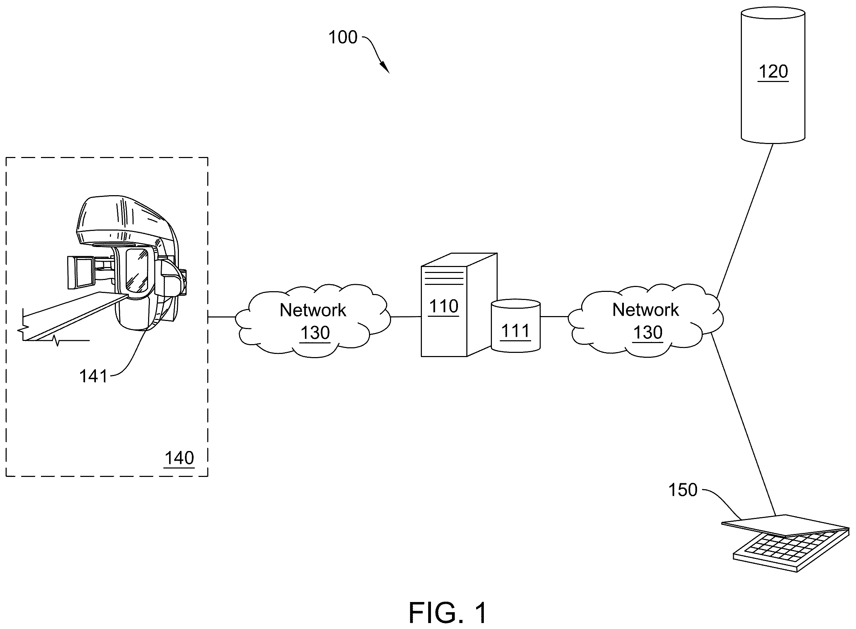

The methods described herein can be implemented to generate the local dose contrast metric using various computing devices/features described in **FIG. 1****.** Therefore, **FIG. 1** describes a non-limiting example of a computer environment where a server can perform the processes/methods described herein, such as retrieving, processing, and presenting radiotherapy treatment data, such as the predicted local dose contrast.

**FIG. 1** illustrates various components of a system **100** for planning and administering SFRT treatments, in accordance with an embodiment. The system **100** may include an analytics server **110,** a medical records database **120,** a radiotherapy system **140,** and a system administrator computer **150** or workstation. These features may communicate with each other over a network **130.** For example, the system **100** may present, via the analytics server **110,** one or more pages/GUIs on the radiotherapy machine **141.** In another example, the system **100** may present, via the analytics server **110,** a page presenting parameters and/or simulated results of an SFRT treatment plan. For example, the analytics server **110** may present a heatmap of radiation dose contrast overlaid on a diagnostic image of a patient to whom the system **100** is planned to administer the SFRT treatment plan.

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a computer-readable, non-transitory storage medium. The processing unit includes a processor with a computer-readable, non-transitory storage medium, such as a random-access memory coupled to the processor. In some embodiments, the analytics server **110** executes algorithms or computer-executable program instructions stored on computer-readable, non-transitory storage medium, which may be executed by a single processor or multiple processors in a distributed configuration. The instructions allow the processor to implement one or more of the functionalities/methods described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, the **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****. In** alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein, such as patient diagnostic images (e.g., x-ray images, computed tomography (CT) scans, magnetic resonance imaging (MRI) scans, ultrasound imaging, positron emission tomography (PET) scans, mammography scans, fluoroscopy scans, endoscopic scans, etc.) and/or operating parameters of the radiotherapy system **140** (e.g., beam energy, penetration depth of the radiation, dose rate, field size, gantry angle, collimator angle, monitor units, treatment time, couch position, beam-on time, etc.). The page may be displayed on a display screen associated with the radiotherapy system **140.**

In some embodiments, the analytics server **110** displays a page showing an SFRT treatment plan dose (e.g., a distribution map, a peak-to-valley dose ratio (PVDR) map, a local dose heterogeneity map, a diagnostic image, etc.). The analytics server **110** may display different pages described herein on a display screen located anywhere within the planning or treatment room, such as located on the wall of the treatment room, any electronic device within the treatment room or any electronic device in the planning room. In some configurations, the analytics server **110** may display the pages on the system administrator computer **150** or workstation.

The local database **111** may also store data corresponding to the radiotherapy machine **141** (e.g., default position of the radiotherapy machine **141,** current position of the radiotherapy machine **141,** such as the orientation of the bed/couch and gantry).

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically prepare and administer the SFRT treatment plan using local dose heterogeneity metrics.

The medical records database **120** may also include the SFRT treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data.

The analytics server **110** may receive treatment data associated with a patient from the medical records database **120,** the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy) or other non-treatment procedures, such as labs and appointments with other medical professionals. The radiotherapy treatment file may include data specific to one or more patients' radiotherapy treatment. The radiotherapy treatment file may include a patient identifier, patient's electronic health data records, medical images (e.g., CT scans, 4D CT Scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index) and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points), the analytics server **110** may execute various image recognition protocols and identify the tumor data.

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system 100. As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

The radiotherapy machine **141** may also include a couch to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies, through the system administrator computer **150** how to align the patient on the couch to optimize the treatment therapy. The radiotherapy machine **141** directs radiation at specified locations of the patient resting on the couch during treatment. The specified locations may be selected by the technician operating the radiotherapy machine **141** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposite the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of a set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can plan and adjust the SFRT treatment plan based on determined local dose heterogeneity. For example, the system administrator computer **150** may be configured to adjust monitor gantry movement, patient information, and/or various thresholds/rules described herein. The system administrator, through the system administrator computer **150,** may plan and adjust various operating parameters of the radiotherapy system **140** such as beam energy, penetration depth of the radiation, dose rate, field size, gantry angle, collimator angle, treatment time, couch position, beam-on time, maximum dose value, and/or minimum dose value, etc. As described herein, in some embodiments, the analytics server **110** automatically adjusts one or more operating parameters based on the determined local dose heterogeneity satisfying or not satisfying a threshold.

The system administrator computer **150** may be configurable to display certain analytics metrics when specific thresholds/rules have been exceeded. For example, the system administrator computer **150** may be alerted if the local dose heterogeneity exceeds a threshold at an organ at risk. Additionally, or alternatively, the system administrator computer **150** may be alerted by the analytics server **110** if the local dose heterogeneity fails to satisfy a threshold at the target tissue.

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

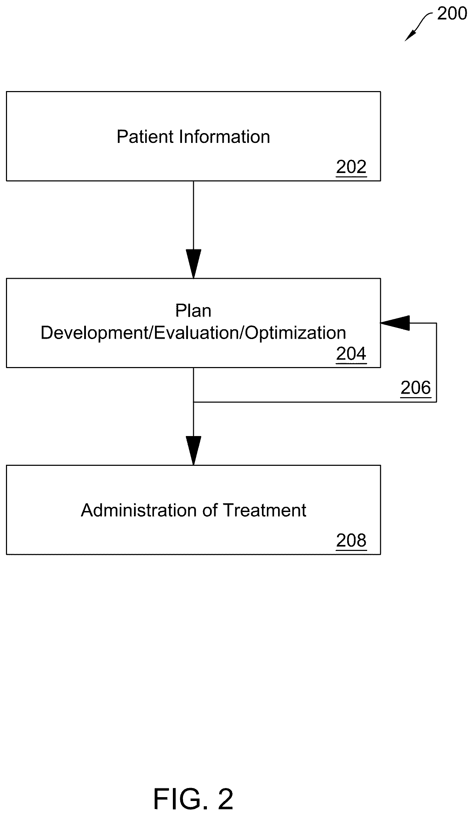

**FIG. 2** is a block diagram illustrating an example of an automated heterogeneous radiotherapy treatment planning process **200** that may be executed by one or more components of the system **100** (e.g., the analytics server **110)** according to the present disclosure. The automated heterogeneous radiotherapy treatment planning process **200,** in whole or in part, may be implemented as a software program, hardware logic, or a combination thereof on/using the system **100** of **FIG. 1****.**

In block **202,** three-dimensional (3D) images of a patient are obtained, and organs and other structures in the patient (the patient geometry) can be segmented and contoured. In block **204** and path **206,** the information from block **202,** and other information such as that mentioned above, are used to develop and evaluate a candidate treatment plan.

In optional block **208,** if the candidate treatment plan is satisfactory (e.g., if it satisfies clinical goals, thresholds, limits), then the plan is administered to the patient. If not, aspects of the treatment plan and/or of the clinical goals may be modified iteratively until a satisfactory plan is generated, such as shown by path 206. For example, one or more treatment parameters of the radiotherapy treatment plan may be adjusted by the analytics server until the satisfactory plan is generated. Treatment parameters may include but are not limited to, beam energy, penetration depth of the radiation, dose rate, field size, gantry angle, collimator angle, monitor units, treatment time, couch position, couch orientation, and/or beam-on time, etc.). Though several treatment parameters are presented above, it is understood that more treatment parameters may exist or be adjusted when generating the satisfactory treatment plan.

The clinical goals may be expressed in terms of, for example, a set of quality metrics, such as conformity to the treatment target, critical organ sparing, and/or target dose contrast, and the like, with respective target values for the quality metrics. A local dose contrast may be generated, graphed, and/or represented to evaluate a treatment plan to determine whether the treatment plan satisfies clinical goals. For example, a treatment plan may be considered to be satisfactory if a local dose contrast of the plan satisfies a specified dose threshold for a specified percentage of the target volume and/or surrounding tissue.

At optional block **208,** when the plan is determined to be satisfactory, various controls (e.g., control signals) may be transmitted to one or more devices to facilitate administration of the radiotherapy treatment plan. For example, the analytics server **110** may transmit control signals to the radiotherapy system **140** to adjust one or more operating parameters of the radiotherapy machine **141** or the radiotherapy system **140** more generally (e.g., beam energy, penetration depth of the radiation, dose rate, field size, gantry angle, collimator angle, monitor units, treatment time, couch position, couch orientation, and/or beam-on time, etc.). Once the operating parameters corresponding to the satisfactory radiotherapy treatment plan are set, the analytics server **110** may transmit instructions to administer the radiotherapy treatment plan by operating the radiotherapy machine. In some embodiments, the analytics server **110** receives an indication (e.g., a confirmation) from the system administrator computer **150** to initiate administration of the SFRT treatment plan.

As shown in path **206,** developing, evaluating, and/or optimizing the radiotherapy treatment plan may be an iterative process. For example, as described in greater detail below, the analytics server **110** may use the ingested information above to generate a local dose contrast to represent the heterogeneous dose at a local position (e.g., a specific position) within a patient volume. The generated local dose contrast may be compared against a threshold to determine the effectiveness of the SFRT treatment plan on the patient and/or damage to the tissue surrounding the target volume.

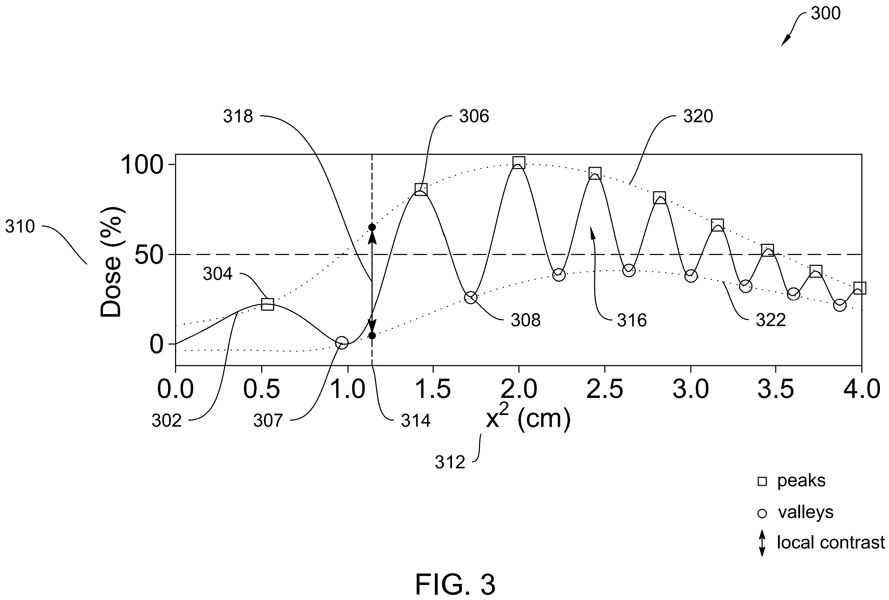

Turning now to **FIG. 3****,** an SFRT treatment plan **300** is shown which may be used to determine a local dose contrast or a heterogeneous dose. The SFRT treatment plan **300** may be defined and/or planned on an electronic device such as the system administrator computer **150** of **FIG. 1****.** In some embodiments, the analytics server **110** of **FIG. 1** may be used to plan, evaluate, and/or optimize the SFRT treatment plan **300.** The SFRT treatment plan **300** may include a nonuniform radiotherapy dose (shown as dose **302)** to apply to a target volume (e.g., a tumor), creating a pattern of high-dose regions (e.g., shown as local maximum dose **304, 306)** and low-dose regions (e.g., shown as local minimum dose **307, 308).** The SFRT treatment plan **300** may be administered by one or more SFRT methods, such as GRID therapy, lattice therapy, and/or Microbeam/Minibeam Radiation Therapy (MRT/MBRT).

In embodiments in which the SFRT treatment plan **300** defines a GRID therapy, a perforated collimator or blocking grid is used to create a pattern of high-dose and low-dose regions. The pattern created by the blocking grid mimics a checkerboard and allows substantial dose escalation in the tumor while sparing surrounding healthy tissues, making it particularly useful for large, bulky tumors that are challenging to treat with conventional radiotherapy. Lattice therapy expands the methods described above in regard to GRID therapy by creating a three-dimensional dose distribution by using multiple perforated collimators or blocking grids. Using advanced imaging and treatment planning systems, lattice therapy targets specific tumor areas with high radiation doses while leaving intervening regions at lower doses, enabling personalized treatment plans tailored to the tumor's size, shape, and location.

Microbeam Radiation Therapy (MRT) and Minibeam Radiation Therapy (MBRT) utilize extremely narrow, high-dose radiation beams to maximize tumor control while sparing normal tissues, such as tissue neighboring the target tissue. In some embodiments, the MRT employs beams with widths in the micron range (e.g., 50 micrometers) whereas MBRT uses slightly larger beams, generally in the 0.5 mm range. These techniques rely on the principle that very small, well-separated high-dose regions allow normal tissues to recover more effectively than with conventional radiation, reducing toxicity and side effects. The primary advantage of MRT and MBRT is their ability to deliver extremely high peak doses to the tumor while keeping the dose in surrounding tissue low, leveraging the biological response known as the "dose-volume effect," where small-scale tissue structures can tolerate high doses better when spatially fractionated.

As described herein, the MRT may employ synchrotron-generated X-rays, which produce highly collimated, parallel beams with ultra-high dose rates. These microbeams create a pattern of local maximum dose **304, 306** and local minimum dose **307, 308,** reducing damage to healthy tissues while maintaining effective tumor control. MBRT, which uses slightly wider beams, has been explored in clinical applications, particularly in settings where high precision is required, such as brain tumors and pediatric cancers.

The SFRT treatment plan **300** shown in **FIG. 3** is illustrated as a graph plotting a dose percentage **310** relative to a position **312.** The dose percentage **310** relates to the percentage of radiation administered (or to be administered) relative to a maximum application (e.g., 100%). As shown in **FIG. 3****,** the dose percentage **310** may be quantified on a scale from 0-100%, with 0% representing no dose and 100% representing full dose. However, it is understood that alternative conventions may be used in which the dose percentage **310** may be quantified on a different scale, such as from 0-1, -1-1, etc. The position **312** may identify a location exposed to the dose **302** (e.g., a radiation beam) during application of the SFRT treatment plan **300.** For example, the position **312** may relate to a position as measured from the collimator, a position as measured from the outer tissue of the patient, etc. As illustrated in **FIG. 3****,** the position **312** is measured in centimeters (cm), however, it is understood that the position **312** may be measured in any suitable distance metric (e.g., millimeters, microns, inches, etc.).

Due to the dynamically oscillating nature of the dose **302,** traditional methods of quantifying the dose **302** are ineffective at precisely defining and measuring the effectiveness of the dose **302** at a particular, local position (e.g., position **314).** For example, traditional metrics for quantifying heterogeneous doses (e.g., the dose **302)** are numerically unstable (e.g., they become undefined or unstable when the dose in the valley or lower dose is **0),** lack local precision, and/or do not account for inconsistent heterogeneities through the patient volume. Thus, a local dose contrast **318** may be generated for the SFRT treatment plan **300** at one or more precise locations to determine, more accurately, the effect (both positive and negative) of the SFRT treatment plan 300 on the patient.

To generate the local dose contrast **318,** the analytics server receives treatment parameters of the SFRT treatment plan **300** to administer to a target tissue (e.g., a tumor). These treatment parameters may be received from the system administrator computer **150 of** **FIG. 1** (e.g., inputs by a medical practitioner at the system administrator computer **150)** and may define a heterogeneous dose including maximum doses and minimum doses of the heterogeneous dose over a distance/volume. The SFRT treatment plan may include a plurality of maximum doses (e.g., local maximum dose **304, 306)** and a maximum of minimum doses (e.g., local minimum dose **307, 308).** The local maximum dose **304,** 306 is a dose that has a higher value (e.g., dose percentage) than all nearby points on the dose **302** within a certain range. The local minimum dose **307, 308** is a dose that has a lower value (e.g., dose percentage) than all nearby points on the dose **302** within a certain range.

Upon receiving the plurality of local maximum doses, the analytics server generates an upper heterogenic curve **320** based at least in part on the plurality of local maximum doses. The analytics server may interpolate between adjacent local maximum doses (e.g., the local maximum dose **304, 306)** to generate the upper heterogenic curve **320.** The interpolation may be any number of methods for interpolation between adjacent local maximum doses, including, but not limited to, linear interpolation, cubic spline interpolation, nearest-neighbor interpolation, bicubic interpolation, Kriging, radial basis function interpolation, Gaussian process regression, inverse distance weighting (IDW), B-spline interpolation, and/or polynomial interpolation. The chosen interpolation method may be used to generate a function that includes one or more of the local maximum doses (e.g., local maximum dose **304, 306).** It is noted that the upper heterogenic curve **320** is based at least in part on the plurality of local maximum doses, but may be based in part on other functions or parameters, such as the interpolation method used, a distance range, etc.

Upon receiving the plurality local minimum doses, the analytics server generates a lower heterogenic curve **322** based at least in part on the plurality of local minimum doses, including adjacent local minimum doses. The analytics server may interpolate between the adjacent local minimum doses (e.g., the local minimum dose **307, 308)** to generate the lower heterogenic curve **322.** The interpolation may be any number of methods for interpolation between the adjacent local minimum doses, including, but not limited to, linear interpolation, cubic spline interpolation, nearest-neighbor interpolation, bicubic interpolation, Kriging, radial basis function interpolation, Gaussian process regression, inverse distance weighting (IDW), B-spline interpolation, and/or polynomial interpolation. The chosen interpolation method may be used to generate a function that includes one or more of the local minimum doses (e.g., local minimum dose **307, 308).** It is noted that the lower heterogenic curve **322** is based at least in part on the plurality of local minimum doses, but may be based in part on other functions or parameters, such as the interpolation method used, a distance range, etc.

The upper heterogenic curve **320** and the lower heterogenic curve **322** provide an upper and lower bound for a dose envelope **316.** Upon generating (or otherwise determining) the upper heterogenic curve **320** and the lower heterogenic curve **322,** the analytics server determines the dose envelope **316.** The dose envelope **316** represents the range of dose variations within the treatment volume, as bounded by the upper heterogenic curve **320** and lower heterogenic curve **322.** Indeed, the dose envelope **316** is defined/bounded at the upper bound by the upper heterogenic curve **320** and defined/bounded at the lower bound by the lower heterogenic curve **322.** By interpolating between the local maximum doses and the local minimum doses to generate the upper heterogenic curve **320** and the lower heterogenic curve **322** (and by extension, the dose envelope **316),** the analytics server may generate and/or predict a local dose contrast for each location within the dose envelope **316.** Thus, the analytics server may generate a local dose contrast to determine a contrast between the upper heterogenic curve **320** and the lower heterogenic curve **322** at any location within the treatment area, enabling graphical and numerical representation/analysis of the dose envelope **316** at any position within the treatment area.

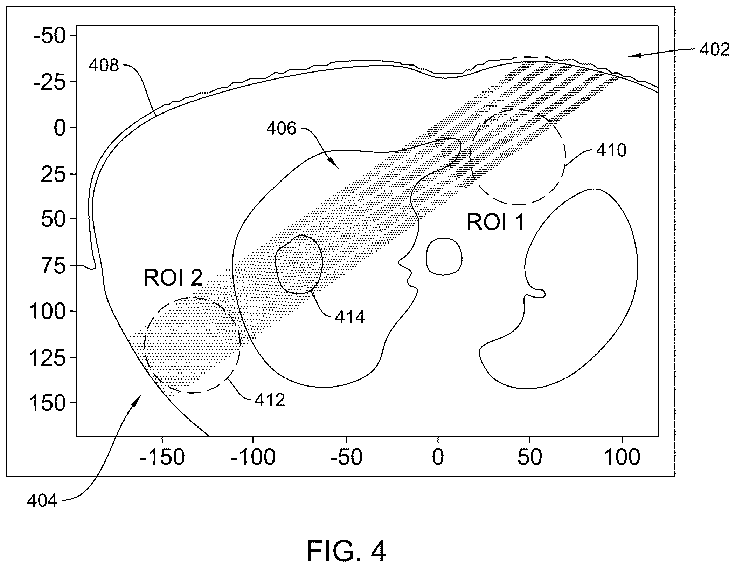

After determining the upper heterogenic curve **320** and the lower heterogenic curve **322,** the analytics server generates a local dose contrast **318** for one or more positions within the dose envelope **316.** This process may be reiterated multiple times to generate the local dose contrast distribution at multiple (in some instances, every) positional point within the treatment volume. The local dose contrast **318** may be graphically illustrated on graphical illustrations of slices of the treatment volume, such as shown in **FIGS. 4-6****,** as described in greater detail below.

The analytics server generates the local dose contrast **318** by calculating the following formula: $γ \left(x\right) = \frac{{D}_{peak} \left(x\right) - {D}_{valley} \left(x\right)}{{D}_{peak} \left(x\right) + {D}_{valley} \left(x\right)}$

In the formula above, γ(x) represents the local dose contrast **318** at the position of interest (e.g., x). *Dₚₑₐₖ*(*x*) represents the value of the upper heterogenic curve **320** at the position of interest. *D_{valley}*(*x*) represents the value of the lower heterogenic curve **322.**

The generated local dose contrast **318 can** be used to drive optimization of the SFRT treatment plan **300,** such as shown by block **204** and path **206** in **FIG. 2****.** When only the weights of the fluence bixels *wⱼ* are optimized, the goal of the optimization algorithm is to minimize a so-called objective function *f* that is a function of the dose *D.* The fluence bixels may refer to the discrete elements of units of radiation fluence that make up the dose **302.** The gradient of the objective function *∇f* can be written as: $∇ f = \frac{\partial f}{\partial {w}_{j}} = \frac{\partial f}{\partial {D}_{i}} ⋅ \frac{\partial {D}_{i}}{\partial {w}_{j}}$

*Dₚₑₐₖ* and *D_{valley}* can be expressed as a function of the dose *D:* $\begin{matrix}{D}_{peak} = {h}_{1} \left(D\right) \\ {D}_{valley} = {h}_{2} \left(D\right)\end{matrix}$

where *h*₁ is for instance a softmax function. *h*₁ and *h*₂ can be chosen arbitrarily, but if both functions are differentiable, the derivative of the local dose contrast **318** (e.g. "y") with respect to the fluence can also be calculated analytically and used directly to drive the optimization: $\frac{\partial γ}{\partial {D}_{i}} = \frac{2 ⋅ \left({h}_{1}′{h}_{2}-{h}_{2}′{h}_{1}\right)}{{\left({h}_{1}+{h}_{1}\right)}^{2}} .$

Once the SFRT treatment plan **300** has been optimized, as described herein, the local dose contrast **318** (or the local dose contrast distribution) may be graphically illustrated as a heatmap, such as shown in **FIGS. 4-6****.** The analytics server may generate a plurality of local dose contrasts to combine together to generate the local dose contrast distribution heatmap of the local dose contrasts. The heatmap of the plurality of the local dose contrasts illustrates the heterogeneity of the radiotherapy treatment plan with the heterogeneous dose of radiation. The heatmap may be color-coded to quickly and efficiently show the heterogeneity of the dose. For example, a higher heterogeneity may be illustrated in red while a lower level of heterogeneity may be illustrated in blue, with a gradient of colors spanning between the higher heterogeneity red and the lower heterogeneity blue. Though red and blue are described herein, it is understood that other colors, shapes, patterns, etc. may be used to indicate higher and lower levels of heterogeneity (e.g., the local dose contrast) of the dose. The heatmap may be graphically overlaid onto a diagnostic image of the patient who will be receiving the radiotherapy treatment. For example, the heatmap may be overlaid onto a PET scan of the patient.

For example, the analytics server may receive the diagnostic image of the patient and the target tissue. The analytics server may display the diagnostic image on a display screen for viewing by a user (e.g., a medical practitioner, technician, patient, etc.).

**FIG. 4** illustrates a dose distribution **406** in a patient's chest **408** with an entry region **402** and an exit region **404.** As can be seen in **FIG. 4****,** at the SFRT treatment plan travels through the patient's chest **408** to a target volume **414,** the beams of radiotherapy (as illustrated by the dose distribution **406)** are scattered, leading to a higher heterogeneity at the entry region **402** than at deeper locations within the patient, such as at the exit region **404.** As shown in **FIG. 4****,** the density of the dots in the dose distribution **406** corresponds to the dose percentage (e.g., from **0-100%),** with the higher density (such as illustrated near the entry region **402)** having a higher dose percentage than a lower density (such as illustrated near the exit region **404).**

The dose distribution **406** allows for an analysis of the radiation dosage of the SFRT treatment plan to better adjust the radiotherapy treatment plan to minimize damage to non-target tissue. For example, the local dose contrast distribution **406** at the first region of interest **410** is shown as having a higher local dose than the second region of interest **412.** However, a local dose contrast between the upper and lower dose values may provide additional useful information for optimizing the SFRT treatment plan.

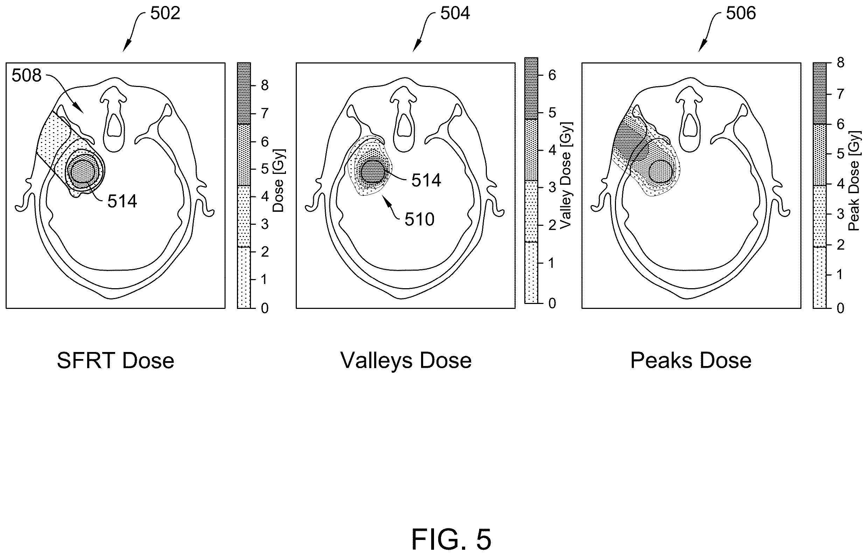

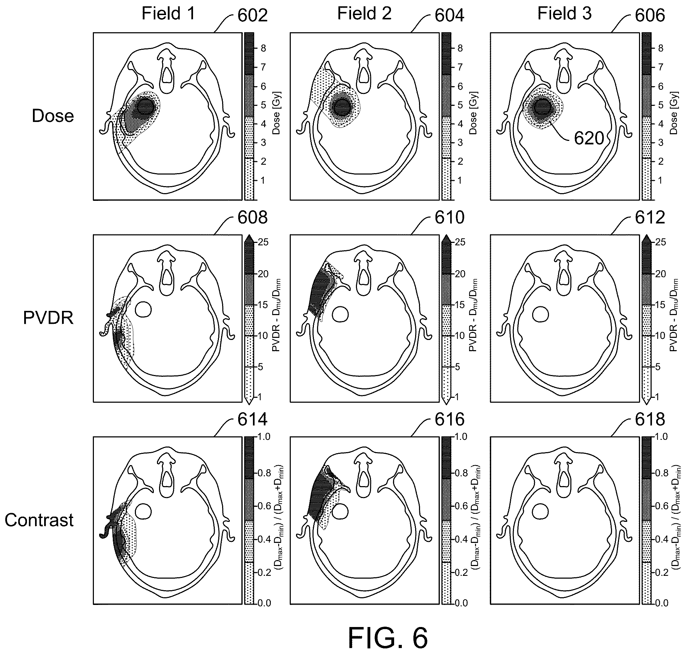

**FIG. 5** illustrates three distinct dose views, including a local dose contrast of the SFRT treatment plan. View **502** illustrates the dose distribution **508** of the SFRT treatment plan when applied to the patient's cranium. As can be seen, the maximum dose distribution is shown at the target volume **514.** View **504,** which shows a graphical illustration of a lower heterogenic curve **510** of the dose distribution **508,** also shows the maximum dose at the target volume **514.** View **506** shows a graphical illustration of an upper heterogenic curve of the SFRT treatment plan, also showing the maximum dose at the target volume **514.** While these three views **502, 504, 506** show the maximum dose being applied to the target volume **514,** they don't show the local dose contrast at the target volume **514,** which shows the effectiveness of the SFRT treatment plan. View **504** and view **506** may be used to generate a local dose contrast distribution heatmap to graphically overlay on the diagnostic image of the patient, such as shown in **FIG. 6****.**

**FIG. 6** illustrates nine distinct views of the SFRT treatment plan, including three dose distribution views (view **602, 604, 606)** showing the dose distribution in a first field (view **602),** a second field (view **604),** and a third field (view **606).** **FIG. 6** illustrates a peak-to-valley dose ratio view in the first field (view **608),** the second field (view **610),** and the third field (view **612).** **FIG. 6** also illustrates a local dose contrast in the first field (view **614),** the second field (view **616),** and the third field (view **618).** As can be seen, all nine views **602, 604, 606, 608, 610, 614, 616, 618** illustrate the same SFRT treatment plan, however, the information provided by the distinct views is different. While view **606** shows a maximum dose distribution at a target volume **620,** the view **618** (which shows the local dose contrast) shows no local dose contrast at all. This may lead to an unsuccessful or ineffective treatment. Likewise, the peak-to-valley dose ratio views (e.g., view **608, 610, 612)** may illustrate the peak-to-valley dose ratio (e.g., calculated by dividing the lower heterogenic curve by the upper heterogenic curve), but fail to provide accurate dose information when the upper or lower heterogenic curve is equal to 0.

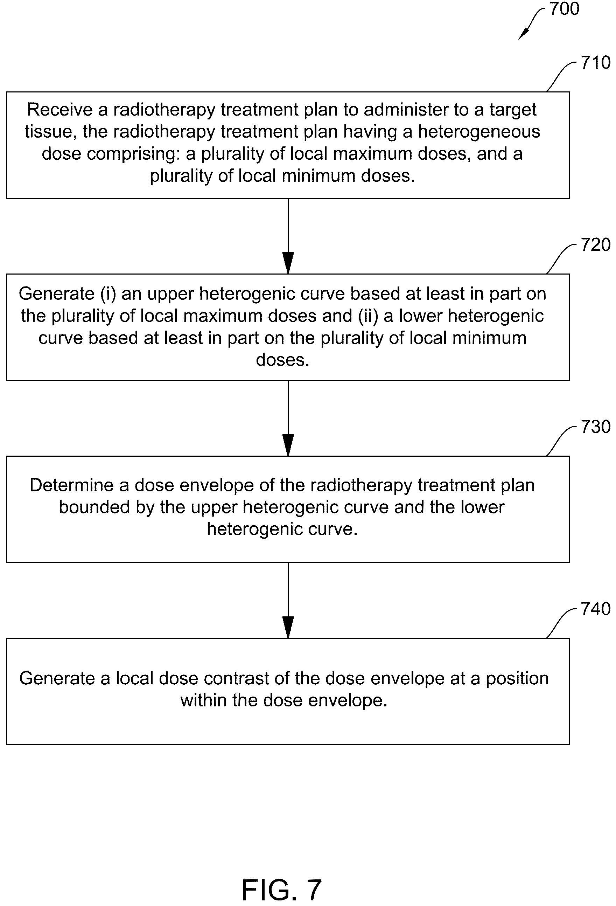

**FIG. 7** **is** a flowchart of an example method **700** for planning and administering an SFRT treatment plan. Though several steps are shown, it is understood that the method **700** may include fewer, more, or different steps than those shown in **FIG. 7****.** The order of the steps in method **700** are illustrated in **FIG. 7** **for** illustrative purposes only. It is understood that the steps of method **700** may be executed in a different order than that shown in **FIG. 7****.**

At step **710,** one or more processors receive a radiotherapy treatment plan to administer to a target tissue, the radiotherapy treatment plan having a heterogeneous dose that includes a plurality of local maximum doses, and a plurality of local minimum doses. The radiotherapy treatment plan may include various treatment parameters of the radiotherapy treatment plan (e.g., dose distribution, beam modality, beam energy, fractionation scheme, grid or lattice pattern, dose prescription, treatment planning system (TPS) parameters, collimation technique, treatment field size, isocenter positioning, patient immobilization, and/or image guidance protocols) and operating parameters of the radiotherapy machine (e.g., beam energy, dose rate, gantry angle, collimator angle, field size, monitor units (MU), leaf sequencing pattern in the multileaf collimator (MLC), beam-on time, pulse repetition frequency, couch position, movement, real-time imaging parameters, calibration settings, quality assurance checks, and/or system software parameters). The radiotherapy treatment plan may also include patient information (e.g., Patient ID, name, date of birth, age, gender, diagnosis, tumor location, treatment plan ID, prescribed dose, fractionation schedule, imaging data, prior treatments, comorbidities, allergies, and/or medications).

At step **720,** the one or more processors generate (i) an upper heterogenic curve based at least in part on the plurality of local maximum doses and (ii) a lower heterogenic curve based at least in part on the plurality of local minimum doses. In various embodiments, the upper heterogeneous curve is generated by interpolating between adjacent maximum doses. In at least one embodiment, the interpolation is a linear interpolation, though any method of interpolation may be used. Likewise, the lower heterogenic curve is generated by interpolating between adjacent minimum doses. By doing so, an upper bound (e.g., the upper heterogenic curve) and a lower bound (e.g., the lower heterogenic curve) are generated for defining a dose envelop of the radiotherapy treatment plan. At step **730,** one or more processors determine the dose envelope of the radiotherapy treatment plan bounded by the upper heterogenic curve and the lower heterogenic curve.

At step **740,** one or more processors generate a local dose contrast (or a plurality of local dose contrasts) of the dose envelope at a position within the dose envelope. As described herein, the local dose contrast may represent the contrast between the upper and lower bounds of the dose envelope at a particular position. Various methods may be used to determine the local dose contrast. In one embodiment, the local dose contrast is determined by dividing the difference of the upper and lower bounds at the position by the sum of the upper and lower bounds at the position. As described above, several local dose contrasts may be generated for various positions within the bounded dose envelope and used to generate a heatmap of the local dose contrast distribution of the plurality of local dose contrasts.

Upon generating the local dose contrast for the dose envelope, the one or more processors may adjust one or more treatment parameters of the spatially fractionated radiotherapy treatment plan. The treatment parameters may include dose parameters associated with the treatment therapy which may include, but are in no way limited by, a dose amount, a dose amplitude, beam modality, beam energy, dose prescription, fractionation scheme, treatment field size, isocenter positioning, collimation technique, multileaf collimator (MLC) configuration, grid or lattice pattern, Peak-to-Valley Dose Ratio (PVDR), treatment planning system (TPS) optimization settings, image guidance protocol, patient immobilization method, beam delivery angle, and/or beam angle. In some embodiments, the treatment parameters include physical operating parameters of a radiotherapy machine that administers the spatially fractionated radiotherapy treatment plan. Physical operating parameters of the radiotherapy machine may include, but are in no way limited to, beam energy, dose rate, gantry angle, collimator angle, field size, multileaf collimator (MLC) leaf positions, beam-on time, pulse repetition frequency, source-to-axis distance (SAD), source-to-skin distance (SSD), treatment couch position, couch rotation, imaging system settings, mechanical isocenter accuracy, accelerator magnet current, cooling system status, and/or power supply settings. Adjusting the one or more treatment parameters may include transmitting one or more control signals to the radiotherapy machine which cause actuation of one or more actuators of the radiotherapy machine.

In some embodiments, the local dose contrast at a location may trigger one or more actions. For example, a target threshold for local dose contrast may set a lower limit for the local dose contrast at a target tissue (e.g., a tumor). In such embodiments, if a target volume at the position does not have a local dose contrast that satisfies (e.g., exceeds) the target threshold, the analytics server may adjust one or more operating or treatment parameters to increase the local dose contrast at the target tissue. Likewise, a threshold for non-target tissues may set an upper limit for the local dose contrast at non-target tissues (e.g., neighboring tissues). In response to the local dose contrast at the non-target tissue exceeding the threshold, the analytics server may adjust one or more operating or treatment parameters to reduce the local dose contrast.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory storage medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM, or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting with the true scope and spirit being indicated by the following claims.

## Claims

1. A method for adjusting a spatially fractionated radiotherapy treatment plan, the method comprising:
receiving, by one or more processors, a radiotherapy treatment plan to administer to a target tissue, the radiotherapy treatment plan having a heterogeneous dose comprising:
a plurality of local maximum doses, and
a plurality of local minimum doses;
generating, by the one or more processors, (i) an upper heterogenic curve based at least in part on the plurality of local maximum doses and (ii) a lower heterogenic curve based at least in part on the plurality of local minimum doses;
determining, by the one or more processors, a dose envelope of the radiotherapy treatment plan bounded by the upper heterogenic curve and the lower heterogenic curve; and
generating, by the one or more processors, a local dose contrast of the dose envelope at a position within the dose envelope, wherein the one or more processors are configured to adjust a treatment parameter of the radiotherapy treatment plan based at least in part on the generated local dose contrast.

2. A system for adjusting a spatially fractionated radiotherapy treatment plan, the system comprising:
one or more processors; and
a computer-readable, non-transitory storage medium containing instructions that, when executed by the one or more processors, cause the one or more processors to perform a method comprising:
receiving a radiotherapy treatment plan to administer to a target tissue, the radiotherapy treatment plan having a heterogeneous dose comprising:
a plurality of local maximum doses, and
a plurality of local minimum doses;
generating (i) an upper heterogenic curve based at least in part on the plurality of local maximum doses and (ii) a lower heterogenic curve based at least in part on the plurality of local minimum doses;
determining a dose envelope of the radiotherapy treatment plan bounded by the upper heterogenic curve and the lower heterogenic curve; and
generating a local dose contrast of the dose envelope at a position within the dose envelope, wherein the one or more processors are configured to adjust a treatment parameter of a radiotherapy treatment plan based at least in part on the generated local dose contrast.

3. The method of claim 1 or the system of claim 2, wherein the method further comprises:
in response in part to the local dose contrast exceeding a threshold, adjusting the treatment parameter of the radiotherapy treatment plan to reduce the local dose contrast.

4. The method or system of claim 1, 2 or 3, wherein the method further comprises:
generating a local dose contrast distribution comprising a plurality of local dose contrasts; and
generating a heatmap of a heterogeneity of the radiotherapy treatment plan based on the local dose contrast distribution.

5. The method or system of any one of claims 1 to 4, wherein the local dose contrast is generated based in part on the upper heterogenic curve and the lower heterogenic curve at the position.

6. The method or system of any one of claims 1 to 5, wherein generating the upper heterogenic curve comprises interpolating the heterogeneous dose between adjacent local maximum doses within the plurality of local maximum doses.

7. The method or system of claim 6, wherein the method further comprises linearly interpolating the heterogeneous dose between the adjacent local maximum doses within the plurality of local maximum doses.

8. The method or system of claim 4, wherein the method further comprises:
receiving a diagnostic image of the target tissue;
displaying the diagnostic image of the target tissue on a display; and
overlaying the heatmap on the diagnostic image of the target tissue.

9. The system of any one of claims 2 to 8, wherein the method further comprises:
adjusting one or more operating parameters of a radiotherapy machine in accordance with the radiotherapy treatment plan; and
transmitting instructions to the radiotherapy machine to administer the radiotherapy treatment plan to the target tissue.

10. A computer-readable, non-transitory storage medium containing instructions for adjusting a spatially fractionated radiotherapy treatment plan and, when executed by one or more processors, cause the one or more processors to perform a method comprising:
receiving a radiotherapy treatment plan to administer to a target tissue, the radiotherapy treatment plan having a heterogeneous dose comprising:
a plurality of local maximum doses, and
a plurality of local minimum doses;
generating (i) an upper heterogenic curve based at least in part on the plurality of local maximum doses and (ii) a lower heterogenic curve based at least in part on the plurality of local minimum doses;
determining a dose envelope of the radiotherapy treatment plan bounded by the upper heterogenic curve and the lower heterogenic curve; and
generating a local dose contrast of the dose envelope at a position within the dose envelope, wherein the one or more processors are configured to adjust a treatment parameter of a radiotherapy treatment plan based at least in part on the generated local dose contrast.

11. The computer-readable, non-transitory storage medium of claim 10, wherein the method further comprises:
in response in part to the local dose contrast exceeding a threshold, adjusting the treatment parameter of the radiotherapy treatment plan to reduce the local dose contrast.

12. The computer-readable, non-transitory storage medium of claim 10 or 11, wherein the method further comprises:
generating a local dose contrast distribution comprising a plurality of local dose contrasts; and
generating a heatmap of a heterogeneity of the radiotherapy treatment plan based on the local dose contrast distribution.

13. The computer-readable, non-transitory storage medium of claim 10, 11 or 12, wherein the local dose contrast is generated based in part on the upper heterogenic curve and the lower heterogenic curve at the position.

14. The computer-readable, non-transitory storage medium of any one of claims 10 to 13, wherein the method further comprises the method of any one of claims 5 to 8.

15. The computer-readable, non-transitory storage medium of any one of claims 10 to 14, wherein the method further comprises:
adjusting, by the one or more processors, one or more operating parameters of a radiotherapy machine in accordance with the radiotherapy treatment plan; and
transmitting, by the one or more processors, instructions to the radiotherapy machine to administer the radiotherapy treatment plan to the target tissue.
